(19)

Europäisches
Patentamt
European
Patent Office
Office européen
des brevets

(11) **EP 4 613 200 A1**

(12) **EUROPEAN PATENT APPLICATION**
published in accordance with Art. 153(4) EPC

(43) Date of publication:
**10.09.2025 Bulletin 2025/37**

(21) Application number: **23885642.1**

(22) Date of filing: **26.10.2023**

(51) International Patent Classification (IPC):
***A61B 5/374*** (2021.01)       ***A61B 5/16*** (2006.01)

(52) Cooperative Patent Classification (CPC):
**A61B 5/16; A61B 5/374**

(86) International application number:
**PCT/JP2023/038668**

(87) International publication number:
**WO 2024/095886 (10.05.2024 Gazette 2024/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.10.2022 JP 2022174759**

(71) Applicant: **LIFESCAPES Inc.
Tokyo 106-0047 (JP)**

(72) Inventors:
• **USHIBA, Junichi
Yokohama-shi, Kanagawa 223-8522 (JP)**
• **IWAMA, Seitaro
Yokohama-shi, Kanagawa 223-8522 (JP)**
• **MORISHIGE, Masumi
Yokohama-shi, Kanagawa 223-8522 (JP)**

(74) Representative: **Plougmann Vingtoft a/s
Strandvejen 70
2900 Hellerup (DK)**

(54) **BRAIN WAVE ANALYSIS DEVICE, BRAIN WAVE ANALYSIS PROGRAM, MOTION ASSISTANCE SYSTEM, AND MOTION ASSISTANCE METHOD**

(57) The present invention relates to an electroencephalogram analysis device and an electroencephalogram analysis program, and a movement assistance system and a movement assistance method. An electroencephalogram analysis device (16) includes a signal acquisition unit (40) that acquires a time series of electroencephalographic signals of an analysis subject (12), and a computation unit (58) that obtains an intrinsic frequency correlated with a movement intention of the analysis subject (12) based on a frequency characteristic related to the time series of the electroencephalographic signals of the analysis subject (12) during rest.

*FIG.2*

EP 4 613 200 A1

**Description**

Technical Field

The present invention relates to an electroencephalogram analysis device and an electroencephalogram analysis program, and a movement assistance system and a movement assistance method.

Background Art

In various fields including medical care, health, nursing care, and sports, there has been heretofore known a technique of detecting a motion or a state of an analysis subject with various sensors and analyzing a movement state or the like of the analysis subject using obtained detection data. As an example of this technique, there has been known a brain machine interface which analyzes electroencephalographic signals to identify an intrinsic frequency indicating a movement intention or a brain state of an analysis subject, and then operates a machine in accordance with the signal intensity of the intrinsic frequency included in the electroencephalographic signals (see Non-Patent Literature 1 and the like).

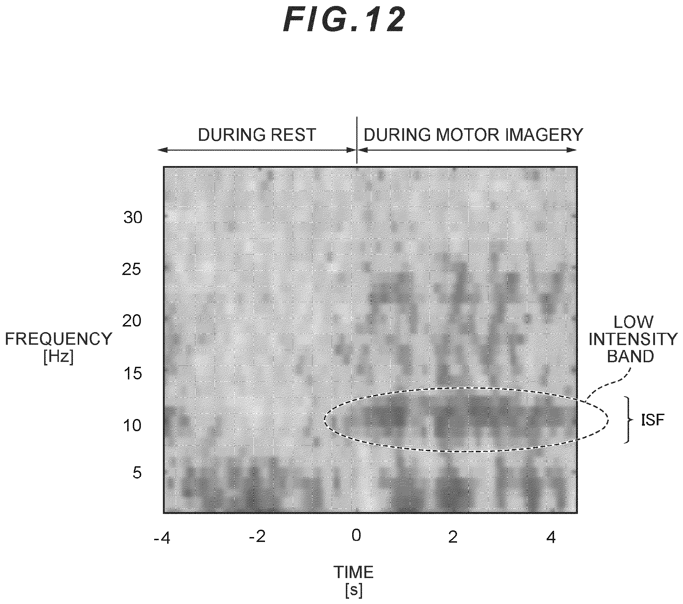

FIG. 12 is a schematic diagram showing a hitherto known measurement method relating to an intrinsic frequency of an analysis subject. The horizontal axis of the map indicates time (unit: s), and the vertical axis of the map indicates frequency (unit: Hz). The "time" herein means an elapsed time for transition from a resting state to a motor imagery state, and the time point at which t = 0 corresponds to a time point of state transition. In addition, the grayscale of the map indicates the magnitude of signal intensity corresponding to a combination of time and frequency, and is defined such that the signal intensity increases as the color becomes lighter.

As can be understood from this diagram, immediately after the transition to the motor imagery state (t > 0), a band-shaped region which extends in the time axis direction and in which the signal intensity is relatively low is generated on the map. The intrinsic frequency corresponding to this band-shaped region is called "individual sensorimotor rhythm event-related desynchronization (SMR-ERD) frequency" (hereinafter, "ISF"), and is known to have high correlation with a movement intention.

Citation List

Non-Patent Literature

Non-Patent Literature 1:"Neurophysiological predictor of SMR-based BCI performance," B. Blankertz et al., NeuroImage, Volume 51, pp. 1303-1309, 2010

Summary of Invention

Technical Problem

However, when the hitherto known measurement method described above is used, in order to increase accuracy in identification of the intrinsic frequency, it is necessary to increase the number of times and duration of "trial" of transition from the resting state to the motor imagery state. As a result, there has been the following problem: not only the time required to identify the intrinsic frequency but also the mental and physical burden on the analysis subject increase.

Specifically, even when a case is assumed where the duration of the resting state is 5 seconds, the duration of the motor imagery state is 5 seconds, and the number of trials is 20, the time required to measure the intrinsic frequency becomes about 3 minutes. In particular, in a case of a patient with a paralyzed part, the time needed to move the part increases, and the measurement time may become 15 minutes, for example. As the measurement time increases, the mental and physical burden on the analysis subject increases.

The present invention has been made in view of such a problem, and an object thereof is to provide an electroencephalogram analysis device and an electroencephalogram analysis program, and a movement assistance system and a movement assistance method, which are capable of significantly reducing the time required for identification when analyzing an electroencephalographic signal of an analysis subject to identify an intrinsic frequency correlated with a movement intention or a brain state.

Solution to Problem

An electroencephalogram analysis device in a first aspect of the present invention includes an acquisition unit that acquires a time series of electroencephalographic signals of an analysis subject, and a computation unit that obtains an intrinsic frequency correlated with a movement intention or a brain state of the analysis subject based on a frequency characteristic related to the time series of the electroencephalographic signals of the analysis subject acquired by the acquisition unit during rest.

An electroencephalogram analysis device in a second aspect of the present invention further includes a calculation unit that calculates a sample value of a peak frequency in the frequency characteristic, and an estimation unit that obtains an estimate value of the peak frequency using a parent population of the sample value calculated by the calculation unit, in which the computation unit transforms the estimate value obtained by the estimation unit into the intrinsic frequency according to a preliminarily set transformation rule.

In an electroencephalogram analysis device in a third aspect of the present invention, the estimation unit obtains the estimate value, and the computation unit transforms the estimate value into the intrinsic frequency; and the intrinsic frequency is thereby obtained without acquiring an electroencephalographic signal of the analysis subject during movement intention.

In an electroencephalogram analysis device in a fourth aspect of the present invention, the estimation unit obtains the estimate value, and the computation unit transforms the estimate value into the intrinsic frequency; and the intrinsic frequency is thereby obtained without requesting the analysis subject to move a paralyzed part.

In an electroencephalogram analysis device in a fifth aspect of the present invention, the estimation unit obtains, for each unit period, the estimate value based on a sequential Bayesian method, using a parent population of the sample value accumulated by repeatedly performing acquisition by the acquisition unit and calculation by the calculation unit for the unit period from a starting point of measurement of the electroencephalographic signals.

An electroencephalogram analysis device in a sixth aspect of the present invention further includes a determination unit that determines, each time the estimation unit performs an estimation, whether a termination condition is met, in which the estimation unit terminates the estimation of the peak frequency when the determination unit determines that the termination condition is met.

In an electroencephalogram analysis device in a seventh aspect of the present invention, the peak frequency is an alpha frequency within an alpha band, the intrinsic frequency is an individual SMR-ERD frequency, and the transformation rule is expressed as an identity function or a linear function with the estimate value as an argument.

In an electroencephalogram analysis device in an eighth aspect of the present invention, the transformation rule is determined depending on the analysis subject.

In an electroencephalogram analysis device in a ninth aspect of the present invention, the acquisition unit acquires either a first time series of the electroencephalographic signals of the analysis subject measured during rest or a second time series of the electroencephalographic signals of the analysis subject measured during motor imagery, and the computation unit performs a first computation in which the intrinsic frequency is obtained using only the first time series.

In an electroencephalogram analysis device in a tenth aspect of the present invention, the computation unit performs the first computation or a second computation in which the intrinsic frequency is obtained using both the first time series and the second time series, with the first computation and the second computation switched.

An electroencephalogram analysis device in an eleventh aspect of the present invention further includes a presentation unit that presents information for requesting a resting state to the analysis subject.

An electroencephalogram analysis device in a twelfth aspect of the present invention further includes an assistance control unit that controls a movement assistance device for assisting a movement of the analysis subject based on the intrinsic frequency obtained by the computation unit.

An electroencephalogram analysis program in a thirteenth aspect of the present invention causes one or more computers to execute: an acquisition step of acquiring a time series of electroencephalographic signals of an analysis subject, and a computation step of obtaining an intrinsic frequency correlated with a movement intention or a brain state of the analysis subject based on a frequency characteristic related to the time series of the electroencephalographic signals of the analysis subject acquired during rest.

A movement assistance system in a fourteenth aspect of the present invention includes: the electroencephalogram analysis device in the twelfth aspect described above; an electroencephalograph that supplies, to the electroencephalogram analysis device, an electroencephalographic signal obtained by measuring an electroencephalogram of an analysis subject; and a movement assistance device that assists a movement of the analysis subject as the movement assistance device operates according to control performed by the electroencephalogram analysis device.

A movement assistance method in a fifteenth aspect of the present invention is a method using a system including an electroencephalograph that measures an electroencephalogram of an analysis subject and outputs electroencephalographic signals, an electroencephalogram analysis device that analyzes the electroencephalographic signals supplied from the electroencephalograph, and a movement assistance device that operates according to control performed by the electroencephalogram analysis device to assist a movement of the analysis subject, the method executing an acquisition step in which the electroencephalogram analysis device measures an electroencephalogram of the analysis subject during rest using the electroencephalograph to acquire a time series of the electroencephalographic signals, a computation step in which the electroencephalogram analysis device obtains an intrinsic frequency correlated with a movement intention or a brain state of the analysis subject based on a frequency characteristic related to the acquired time series of the electroencephalographic signals, a calibration step in which the movement assistance device is subjected to calibration with the obtained intrinsic frequency set as a calibration parameter, and an assistance step in which a movement of the analysis subject is assisted by controlling an operation of the calibrated movement assistance device.

Advantageous Effect of Invention

According to the present invention, when electroencephalographic signals of an analysis subject are analyzed to identify an intrinsic frequency correlated with a movement intention, the time required for identification can be significantly reduced.

Brief Description of Drawings

[FIG. 1] FIG. 1 is an overall configuration diagram of a BMI system into which an electroencephalogram analysis device according to an embodiment of the present invention is incorporated.
[FIG. 2] FIG. 2 is a functional block diagram of the processor and the memory illustrated in FIG. 1.
[FIG. 3] FIG. 3 is a flowchart related to a movement assistance method using the BMI system illustrated in FIG. 1.
[FIG. 4] FIG. 4 is a flowchart related to an analysis operation performed by the electroencephalogram analysis device of FIG. 1.
[FIG. 5] FIG. 5 is a graph showing temporal changes in electroencephalographic signals during rest.
[FIG. 6] FIG. 6 is a graph showing a method of calculating an IAF sample value.
[FIG. 7] FIG. 7 is a graph showing an example of convergence properties of an IAF estimate value according to a sequential Bayesian method.
[FIG. 8] FIG. 8 is a graph schematically showing a time reduction effect of cutoff processing during convergence.
[FIG. 9] FIG. 9 is a diagram illustrating a relationship of ISF to IAF.
[FIG. 10] FIG. 10 is a graph showing a probability density distribution related to deviation between IAF and ISF.
[FIG. 11] FIG. 11 is a schematic diagram illustrating an effect of an electroencephalogram analysis method according to the embodiment.
[FIG. 12] FIG. 12 is a schematic diagram illustrating a hitherto known measurement method relating to an intrinsic frequency of an analysis subject.

Description of Embodiment

An embodiment of the present invention will be described with reference to attached drawings below. In order to facilitate understanding of the description, the same reference numerals are given to the same components and steps in the drawings as much as possible, and redundant description is omitted.

[Configuration of BMI System]

<Overall Configuration>

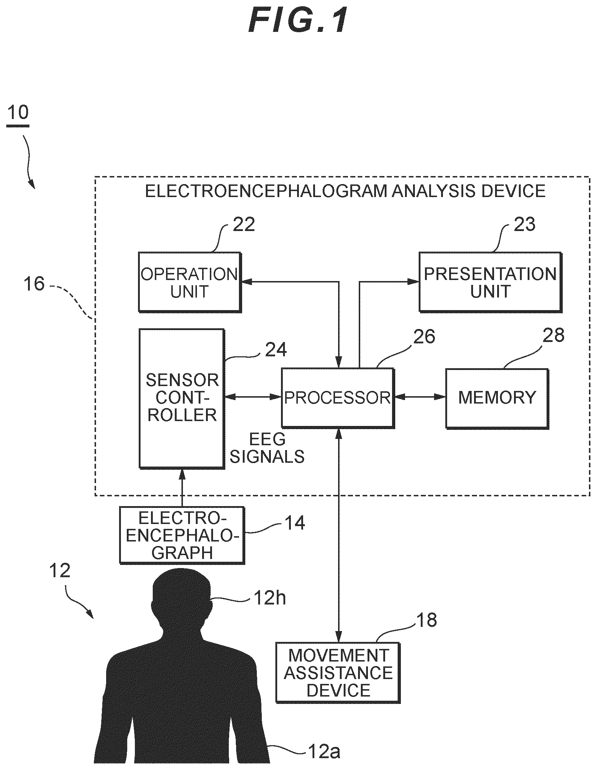

FIG. 1 is an overall configuration diagram of a brain machine interface system (hereinafter, the BMI system 10) into which an electroencephalogram analysis device 16 according to the embodiment of the present invention is incorporated. The BMI system 10 is configured to analyze an electroencephalogram generated by an analysis subject 12 and is configured to be capable of assisting a movement of the analysis subject 12 on the basis of an analysis result thereof. Specifically, the BMI system 10 is configured to include an electroencephalograph 14, an electroencephalogram analysis device 16, and a movement assistance device 18.

The electroencephalograph 14 is, for example, a headset configured to be capable of measuring an electroencephalogram generated in a head 12h of the analysis subject 12. The electroencephalograph 14 outputs an electric signal detected via an electrode (not shown) to the electroencephalogram analysis device 16.

The electroencephalogram analysis device 16 is a computer configured to be capable of analyzing, on the basis of an electroencephalographic signal measured by the electroencephalograph 14, a movement intention of the analysis subject 12 or a brain state such as fatigue or cognition of the analysis subject 12. Specifically, the electroencephalogram analysis device 16 includes an operation unit 22, a presentation unit 23, a sensor controller 24, a processor 26, and a memory 28.

The operation unit 22 is configured to be capable of executing various operations by a user including the analysis subject 12 and a health professional. The operation unit 22 is an input device including an operation button and a microphone, or is an output device including a display panel and a speaker, for example.

The presentation unit 23 is an output device that presents, in response to an instruction from the processor 26, information (hereinafter, also referred to as the request information) for requesting a resting state to the analysis subject 12. The presentation unit 23 is composed of, for example, a display panel, a lamp, a speaker, and the like. Examples of a mode of presenting the request information include guidance by text or voice, lighting of a lamp, and output of various types of sound. Note that the subject presenting the request information is not limited to the presentation unit 23 of the electroencephalogram analysis device 16, and may be a person (for example, an operator of the electroencephalogram analysis device 16) different from the analysis subject 12.

The sensor controller 24 is a control circuit that performs various types of control for the electroencephalograph 14. The sensor controller 24 can execute various types of signal processing including sampling processing including synchronization of sensors, low-pass filtration processing, and A/D conversion processing, for example. Consequently, the sensor controller 24 acquires electric signals (that is, electroencephalographic signals) indicating an electroencephalogram of the analysis subject 12 at a predetermined sampling interval and supplies the electric signals to the processor 26. Specifically, the sampling interval can take any value within a range of several tens to several hundreds of milliseconds.

The processor 26 comprehensively controls each unit constituting the electroencephalogram analysis device 16. The processor 26 may be a general-purpose processor including a central processing unit (CPU) and a micro-processing unit (MPU), or may be a special purpose processor including a field programmable gate array (APGA) and a graphics processing unit (GPU) .

The memory 28 is a non-transitory storage medium including a read only memory (ROM) and a random access memory (RAM), and stores a program and data required for the processor 26 to control each component.

The movement assistance device 18 is a device for assisting or supporting the analysis subject 12 in moving a target part (an arm 12a in the example of this diagram). Examples of the target part include various body parts that perform extension/flexion movements, such as a hand, a foot, a finger, a knee, and an elbow, in addition to the arm 12a. The movement assistance device 18 may be a "wearable robot" that assists, by driving an actuator, the analysis subject 12 in performing extension and flexion movements of a body part, or may be an "illusion-inducing device" that assists, by providing an illusory stimulus through visual or tactile perception, a patient in performing extension and flexion movements of a body part.

<Functional Block>

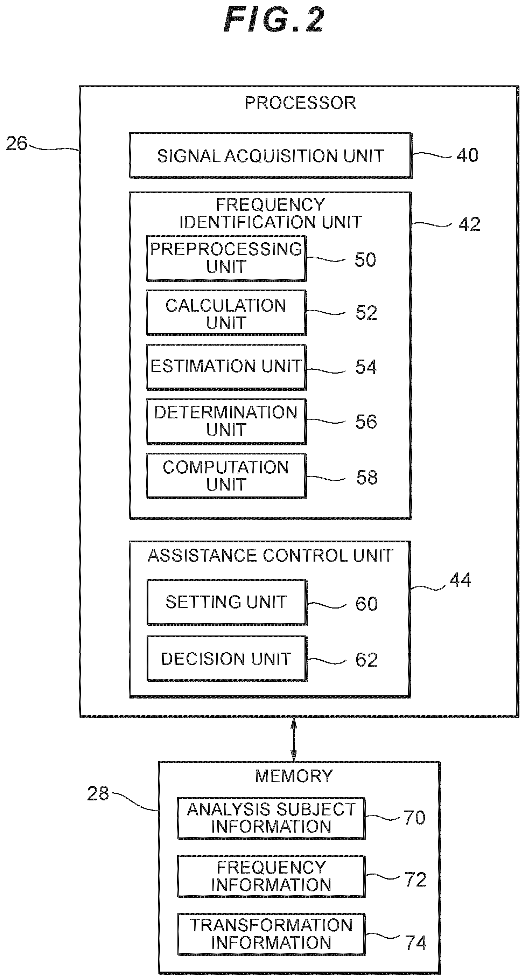

FIG. 2 is a functional block diagram of the processor 26 and the memory 28 illustrated in FIG. 1. The processor 26 functions as a signal acquisition unit 40 (corresponding to "acquisition unit"), a frequency identification unit 42, and an assistance control unit 44 by reading an electroencephalogram analysis program from the memory 28 and executing the electroencephalogram analysis program.

The signal acquisition unit 40 acquires a series of electroencephalographic signals of the analysis subject 12 via the sensor controller 24 (FIG. 1). Consequently, electroencephalographic signals within a unit period are sequentially acquired. The unit period may be a duration equal to the sampling interval or a duration of an integer multiple of the sampling interval. The time series of electroencephalographic signals includes electroencephalographic signals of the analysis subject 12 measured during rest (hereinafter, also referred to as a "first time series") or electroencephalographic signals of the analysis subject 12 measured during motor imagery (hereinafter, also referred to as a "second time series").

The frequency identification unit 42 analyzes the electroencephalographic signals acquired by the signal acquisition unit 40, thereby identifying an intrinsic frequency correlated with a movement intention of the analysis subject 12. Examples of the intrinsic frequency include individual event-related desynchronization frequency (ERD frequency) and individual SMR-ERD frequency (that is, the ISF).

The "SMR-ERD frequency" herein refers to a frequency at which event-related desynchronization (ERD), which is a motor-related response, is most pronounced within the alpha band (8 to 13 Hz) of a scalp electroencephalogram recorded near the motor cortex. It is known that the SMR-ERD frequency varies among individuals and fluctuates within the range of 8 to 13 Hz. Therefore, to explicitly indicate that the frequency is specific to an individual, the frequency is sometimes referred to as individual SMR-ERD frequency (that is, the ISF).

Specifically, the frequency identification unit 42 includes a preprocessing unit 50, a calculation unit 52, an estimation unit 54, a determination unit 56, and a computation unit 58.

The preprocessing unit 50 performs, on the time series of the electroencephalographic signals acquired by the signal acquisition unit 40, preprocessing necessary to calculate an individual alpha frequency (hereinafter, IAF). The preprocessing includes, for example, [1] "filter processing," which involves a moving average, [2] "frequency transformation processing," which includes fast Fourier transform (FFT), and [3] "detrending processing," which removes 1/f noise from a frequency characteristic (or power spectrum).

The "alpha frequency" herein refers to a frequency at which a peak of signal intensity appears within the alpha band of 8 to 13 Hz, among a scalp electroencephalogram reflecting the collective activity of brain neurons. It is known that the alpha frequency varies among individuals and fluctuates within the range of 8 to 13 Hz. Therefore, to explicitly indicate that the frequency is specific to an individual, the frequency is sometimes referred to as individual alpha frequency (that is, the IAF).

The calculation unit 52 calculates the IAF of the analysis subject 12 from the frequency characteristic of the electroencephalographic signals obtained by the preprocessing unit 50, thereby obtaining a sample value of each time series (hereinafter, also referred to as the "IAF sample value"). Specifically, the calculation unit 52 detects the maximum peak within a specific band (the alpha band in this case) in the frequency characteristic and calculates the IAF with the frequency corresponding to the maximum peak taken as the IAF sample value. Note that in addition to the alpha band (8 to 13 Hz), at least one of the delta band (1 to 3 Hz), the theta band (3 to 7 Hz), the beta band (14 to 30 Hz), and the gamma band (30 Hz or more) is selected as the specific band depending on an analysis target.

The estimation unit 54 estimates the IAF of the analysis subject 12 using a parent population of the IAF sample value calculated by the calculation unit 52, thereby obtaining an estimate value (hereinafter, also referred to as the "IAF estimate value") for each parent population. Various statistical methods, including the Bayesian method and the sequential Bayesian method (or the Kalman filter), are used as estimation techniques. For example, the estimation unit 54 may obtain the estimate value based on the sequential Bayesian method for each unit period using a parent population of electroencephalographic signals accumulated from a starting point of measurement.

The determination unit 56 determines whether the IAF estimate value meets a termination condition each time the estimation unit 54 performs an estimation, and instructs the estimation unit 54 to terminate the estimation process when the termination condition is met. Examples of the termination condition include: [Condition 1] the IAF estimate value has converged (for example, a change amount has fallen below a threshold value); [Condition 2] the number of times the estimation was performed by the estimation unit 54 has exceeded a threshold value; [Condition 3] a certain amount of time has elapsed since the starting point of electroencephalographic signal measurement; and [Condition 4] a combination of Conditions 1 to 3 described above.

The computation unit 58 obtains a transformed value (hereinafter, the ISF-transformed value) for each analysis operation by transforming the IAF estimate value obtained immediately when the determination unit 56 determines that the termination condition is met into the intrinsic frequency (ISF in this case) correlated with a movement intention of the analysis subject 12 in accordance with a predetermined transformation rule. The transformation rule may be a rule common to the analysis subject 12, or may be a different rule depending on the analysis subject 12. When the transformation rule is a function that takes the IAF as an argument, the function form may be [1] a linear function such as an identity function or a linear function, or may be [2] a nonlinear function such as a polynomial function with an exponent of 2 or greater, or an exponential function.

Incidentally, the computation unit 58 performs computation processing (hereinafter, referred to as the "first computation") to obtain the ISF using only the first time series described above; however, computation (hereinafter, referred to as the "second computation") may be performed to obtain the ISF using both the first time series and the second time series in conjunction with the first computation. In this case, the computation unit 58 may switch between the first computation and the second computation as necessary. The two types of computation may be switched manually through an input operation from the operation unit 22 (FIG. 1) or may be switched automatically on the basis of an analysis result of the electroencephalographic signals acquired by the signal acquisition unit 40, for example.

The assistance control unit 44 performs control (hereinafter, also referred to as the "assistance control") for the movement assistance device 18 on the basis of the ISF-transformed value obtained by the computation unit 58. The assistance control unit 44 is configured to include a setting unit 60 that sets an ISF setting value suitable for the analysis subject 12, and a decision unit 62 that decides a control amount of the movement assistance device 18 using the frequency characteristic of the electroencephalographic signals and using the ISF setting value set by the setting unit 60.

On the other hand, the memory 28 stores analysis subject information 70, frequency information 72, and transformation information 74 in association with each other.

The analysis subject information 70 includes various pieces of information related to the analysis subject 12, such as identification and personal information on the analysis subject 12, a diagnostic result and recovery status of the analysis subject 12, and the type and usage history of the movement assistance device 18, for example. The frequency information 72 includes various pieces of information related to the peak frequency or the intrinsic frequency, for example, the IAF sample value, the IAF estimate value, and the ISF-transformed value. The transformation information 74 includes various pieces of information enabling identification of the transformation rule, for example, the type, coefficient, and order of the function form, and a look-up table (LUT).

[Operation of BMI System 10]

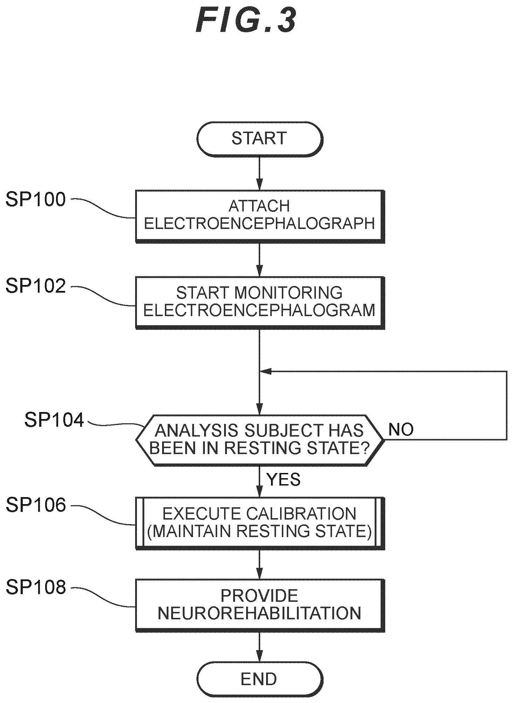

The BMI system 10 in the embodiment is configured as described above. Next, an operation of the BMI system 10, more specifically, a movement assistance operation by the electroencephalogram analysis device 16 will be described with reference to flowcharts of FIGs. 3 and 4 and FIGs. 5 to 11.

<Description of Movement Assistance Method>

FIG. 3 is a flowchart related to a movement assistance method using the BMI system 10 illustrated in FIG. 1. In a step SP100, an "attaching" step of attaching the electroencephalograph 14 on the head of the analysis subject 12 is executed. In a step SP102, a "starting" step of starting monitoring of an electroencephalogram generated by the analysis subject 12 is executed. In a step SP104, a "confirming" step of confirming whether the analysis subject 12 has been in a resting state is executed. When the analysis subject 12 is not in the resting state (step SP104: NO), the process remains in the step SP104 until the analysis subject 12 falls into the resting state. On the other hand, when the analysis subject 12 has been in the resting state (step SP104: YES), the process proceeds to a next step SP106.
In the step SP106, a "calibrating" step of subjecting the movement assistance device 18 to calibration is executed. Specifically, after the step SP102, the electroencephalogram analysis device 16 analyzes the electroencephalographic signals sequentially supplied from the electroencephalograph 14 to obtain an intrinsic frequency (that is, the ISF) of the analysis subject 12, and sets the value of the ISF as a calibration parameter for the movement assistance device 18.
In the step SP108, an "assisting" step of providing neurorehabilitation to the analysis subject 12 is executed. Specifically, the electroencephalogram analysis device 16 controls the operation of the movement assistance device 18 which has been calibrated in the step SP106. Then, through the operation of the movement assistance device 18, a movement of the analysis subject 12 is assisted.

<Analysis Operation by Electroencephalogram Analysis Device 16>

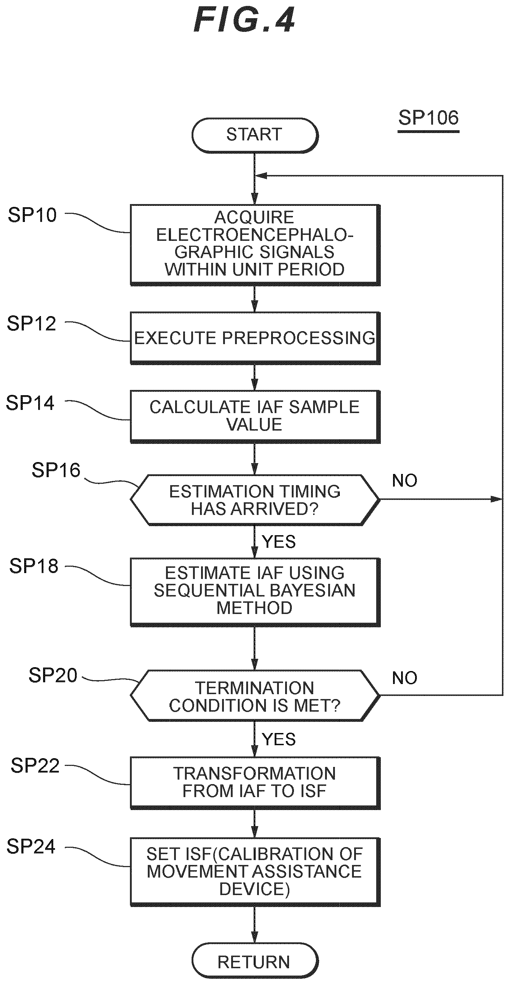

FIG. 4 is a flowchart related to an analysis operation performed by the electroencephalogram analysis device 16 of FIG. 1. For example, the electroencephalogram analysis device 16 executes this flowchart in order to identify the intrinsic frequency (that is, the ISF) of the analysis subject 12 prior to rehabilitation of the arm 12a of the analysis subject 12. This flowchart may be executed not only at the start of rehabilitation and not only once but also one or more times as needed during rehabilitation. In addition, this operation can be directly confirmed by an analysis by an electroencephalogram analysis program and can also be indirectly confirmed by comparing an ideal signal waveform that is input into the electroencephalograph 14 as simulated input with an output result from the electroencephalogram analysis device 16.
In a step SP10 of FIG. 4, the signal acquisition unit 40 acquires a time series of electroencephalographic signals within a unit period via the electroencephalograph 14 and the sensor controller 24.

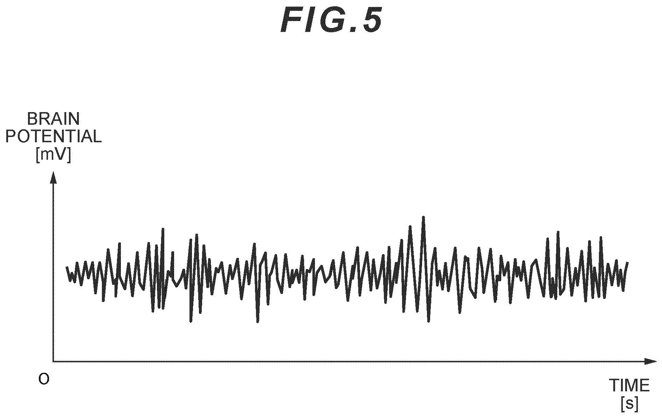

FIG. 5 is a graph showing temporal changes in electroencephalographic signals in a resting state. The horizontal axis of the graph indicates time (unit: s), and the vertical axis of the graph indicates brain potential (unit: mV). As understood from this graph, the electroencephalographic signals form a complex waveform that finely fluctuates up and down around a reference value.
In a step SP12 of FIG. 4, the frequency identification unit 42 (more specifically, the preprocessing unit 50) performs preprocessing on the time series of the electroencephalographic signals acquired in the step SP10. Consequently, a frequency characteristic of the electroencephalographic signals is obtained.
In a step SP14, the frequency identification unit 42 (more specifically, the calculation unit 52) calculates the IAF sample value from the frequency characteristic obtained through the preprocessing in the step SP12.

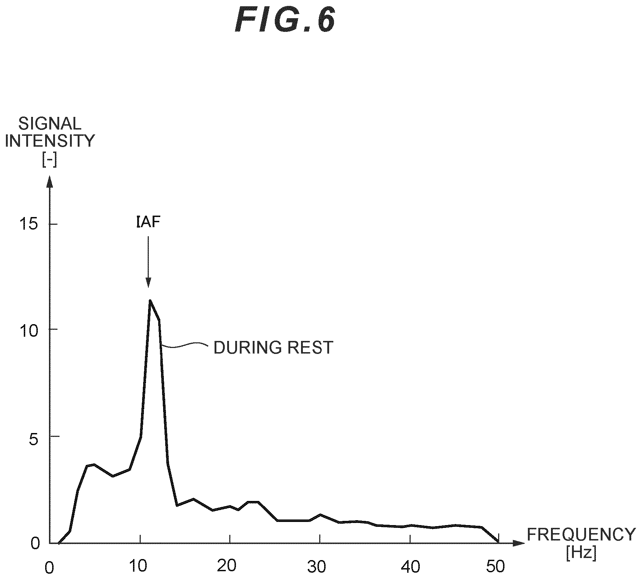

FIG. 6 is a graph showing a method of calculating the IAF sample value. The horizontal axis of the graph indicates frequency (unit: Hz), and the vertical axis of the graph indicates signal intensity (unit: dimensionless). As understood from this graph, the frequency characteristic has a maximum peak around 11 [Hz]. That is, the IAF sample value is calculated as 11 [Hz].
In a step SP16 of FIG. 4, the processor 26 checks whether the timing (hereinafter, referred to as the "estimation timing") at which the IAF is estimated has arrived. When the estimation timing has not yet arrived (step SP16: NO), the processor 26 returns the process to the step SP10 and sequentially repeats the execution of the steps SP10 to SP16 until the timing has arrived. On the other hand, when the estimation timing has arrived (step SP16: YES), the processor 26 advances the process to a next step SP18.
In the step SP18, the frequency identification unit 42 (more specifically, the estimation unit 54) estimates the IAF by applying the sequential Bayesian method to a parent population accumulated through sequential calculation in the step SP14. For example, according to the Bayesian method, a posterior probability p (IAF = Ac|Data) is calculated using a prior probability p (IAF = A) according to Equation (1) below. In the case of the sequential Bayesian method, a current posterior probability is calculated with a previous posterior probability taken as a current prior probability.

[Math. 1]

$$p(IAF = A|Data) = \frac{p(Data|IAF = A)p(IAF = A)}{p(Data)} \quad \cdots (1)$$

In a step SP20, the frequency identification unit 42 (more specifically, the determination unit 56) determines whether the IAF estimate value obtained in the step SP18 meets the termination condition. When the termination condition is not met (step SP20: NO), the processor 26 returns the process to the step SP10 and repeats the execution of the steps SP10 to SP20 sequentially until the termination condition is met.

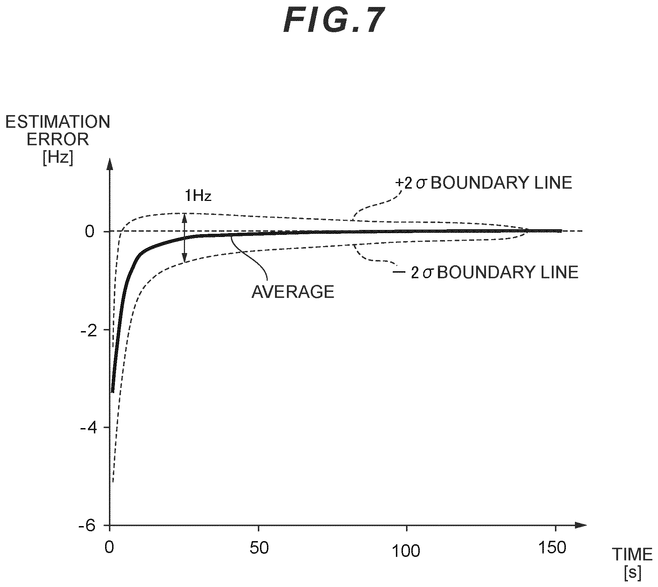

FIG. 7 is a graph showing an example of convergence properties of an IAF estimate value according to the sequential Bayesian method. The horizontal axis of the graph indicates time (unit: s), and the vertical axis of the graph indicates an estimation error (unit: Hz). The "time" herein corresponds to an elapsed time from a starting point (t = 0) of estimation by the sequential Bayesian method. In addition, the "estimation error" corresponds to a value (that is, a deviation) obtained by subtracting an actual value from the IAF estimate value.

The solid curve shows an average value of data of 180 healthy adults. In addition, the lower dashed curve shows a boundary line of "average value - 2σ" (σ: standard deviation), and the upper dashed curve shows a boundary line of "average value + 2σ." As can be understood from this graph, although the estimation error is large at the starting point, the estimation error becomes exponentially small over time, and the estimation error finally converges to zero or a value in the vicinity thereof.

For example, assuming that the behavior of the IAF estimate value follows a Gaussian distribution, setting the estimation time to 26 seconds allows the estimation error to be kept within 1 Hz for 95.7% of healthy adults (that is, within a 4σ range). Compared to a hitherto known measurement method illustrated in FIG. 12 (which takes several minutes to more than ten minutes), the required time is significantly reduced.

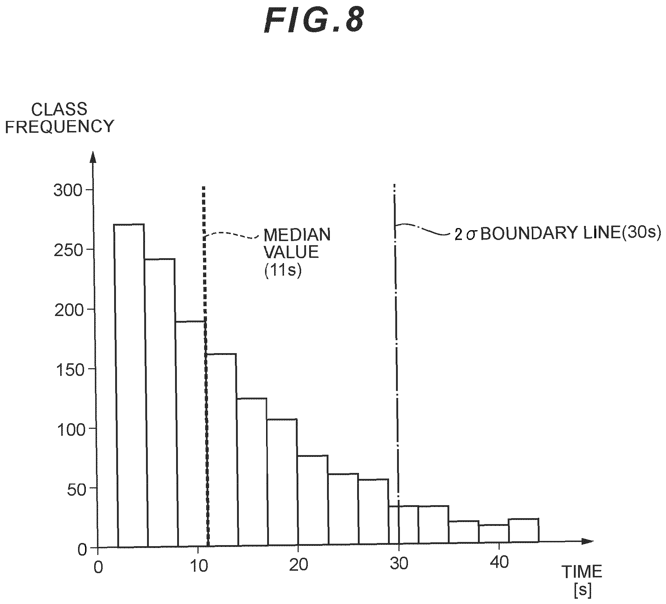

FIG. 8 is a graph schematically showing a time reduction effect of cutoff processing during convergence. An axis of the histogram indicates estimation time required for the estimation error (see FIG. 6) of an individual to fall within 1 Hz, that is, convergence time (unit: s). According to this histogram, the class frequency is highest for the convergence time within 4 seconds, and the class frequency gradually decreases as the convergence time increases.

As understood from this graph, the median value of the convergence time corresponds to "11 s," and the 2σ boundary line of the convergence time corresponds to "30 s." That is, in a case where the estimation error in 95.7% of healthy adults is designed to fall within 1 Hz, it is possible to further reduce about 2/3 (30 - 11 = 19 s) of the estimation time by introducing cutoff processing during convergence ([Condition 1] described above).

On the other hand, when the process returns to the step SP20 of FIG. 4 and the termination condition is met (step SP22: NO), the processor 26 advances the process to a next step SP22.

In the step SP22 of FIG. 4, the frequency identification unit 42 (more specifically, the computation unit 58) transforms the IAF estimate value most recently obtained in the step SP18 into the ISF in accordance with the transformation rule identified from the transformation information 74. Specifically, the ISF is transformed according to Equation (2) below. Note that g(·) is an arbitrary function.

[Math. 2]

$$ISF = g(IAF) \quad \cdots (2)$$

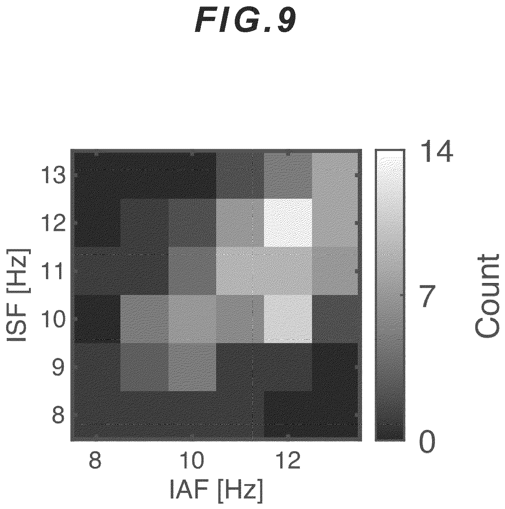

FIG. 9 is a diagram illustrating a relationship of ISF to IAF. This diagram indicates the relationship by grayscale grids on the basis of the number of counts of data pairs of IAF and ISF from 187 healthy adults, and indicates that the lighter the grayscale (closer to white), the higher the number of counts. As understood from the diagram, the ISF has a constant positive correlation (r = 0.62) with the IAF.

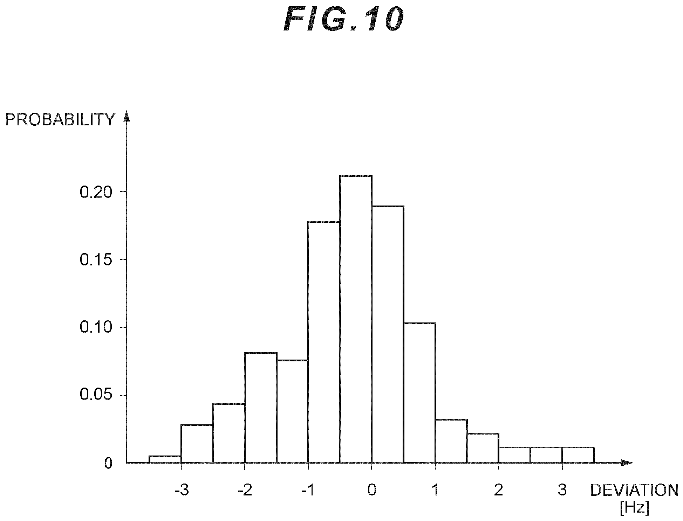

FIG. 10 is a graph showing a probability density distribution related to deviation between IAF and ISF. The "deviation" herein is defined by $\Delta$ = IAF - ISF (unit: Hz). As understood from the graph, the probability in this distribution has a local maximum at the point where deviation $\Delta$ = 0 Hz.

That is, the ISF may be calculated according to Equation (3) below, focusing on the relationships shown in FIGs. 9 and 10. In this case, g(x) = x applies in Equation (2), and so-called identity transformation is represented.

[Math. 3]

$$ISF = IAF \quad \cdots (3)$$

In the step SP22 of FIG. 4, the assistance control unit 44 (more specifically, the setting unit 60) sets the ISF-transformed value obtained in the step SP20. Consequently, the assistance control unit 44 can control the movement assistance device 18 so as to be suitable for the analysis subject 12.

<Effect Provided by Above Method>

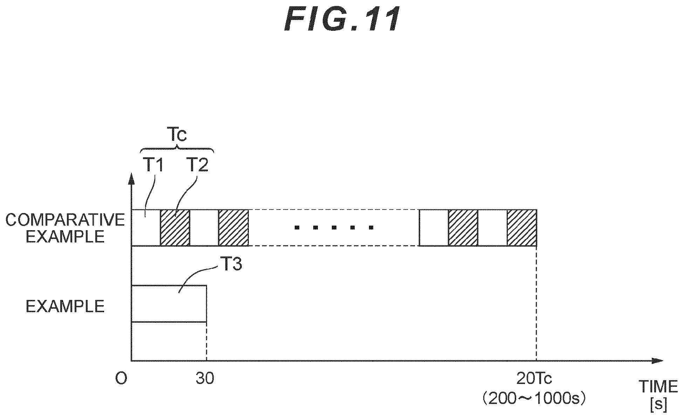

FIG. 11 is a schematic diagram illustrating an effect of an electroencephalogram analysis method in the embodiment. The axis of the graph represents time (unit: s) from the starting point of measurement of an electroencephalogram. The upper bar shows a breakdown of an ISF identification time in a "comparative example" (see FIG. 12). The lower bar shows a breakdown of an ISF identification time in an "example" (see FIGs. 3 and 4).

In a "comparative example," when the time to reach a resting state is taken as T1, the time during which a motor imagery state is maintained is taken as T2, and the number of measurement trials is set to 20, the measurement time for the intrinsic frequency is 20Tc (provided that Tc = T1 + T2). In particular, in a case of the analysis subject 12 with a paralyzed part, the time required to move the part becomes long, and for example, the measurement time may become 200 to 1000 **s.** As the measurement time becomes longer, the mental and physical burden on the analysis subject 12 increases.

On the other hand, in the "example," when the total time required from reaching the resting state, starting estimation of the ISF, and completing the estimation is taken as T3, as already described in FIG. 7 and FIG. 8, T3 becomes about 30 s. That is, the ISF is identified in a short time without acquiring an electroencephalographic signal of the analysis subject 12 during movement intention or without requesting the analysis subject 12 to move the paralyzed part. Consequently, the mental and physical burden on the analysis subject 12 is significantly reduced.

[Summary of embodiment]

As described above, the movement assistance system (the BMI system 10 in this case) in the embodiment includes the electroencephalogram analysis device 16, the electroencephalograph 14 that supplies, to the electroencephalogram analysis device 16, electroencephalographic signals obtained by measuring an electroencephalogram of the analysis subject 12, and the movement assistance device 18 that assists a movement of the analysis subject 12 as the movement assistance device 18 operates according to control performed by the electroencephalogram analysis device 16.

The electroencephalogram analysis device 16 includes an acquisition unit (the signal acquisition unit 40 in this case) that acquires a time series of electroencephalographic signals of the analysis subject 12, and the computation unit 58 that obtains an intrinsic frequency correlated with a movement intention or a brain state of the analysis subject 12 on the basis of a frequency characteristic related to the acquired time series of the electroencephalographic signals.

In addition, according to the electroencephalogram analysis method and the electroencephalogram analysis program in the embodiment, one or more computers (or the processors 26) execute: an acquisition step (SP 10) of acquiring a time series of electroencephalographic signals of the analysis subject 12, and a computation step (SP22) of obtaining an intrinsic frequency correlated with a movement intention of the analysis subject 12 on the basis of a frequency characteristic related to the time series of the electroencephalographic signals of the analysis subject 12 acquired during rest.

In addition, according to the movement assistance method and the movement assistance program in the embodiment, one or more computers (or the processors 26) further execute, in addition to the acquisition step (SP 10) and the computation step (SP22) described above: a calibration step (SP24) in which the movement assistance device 18 is subjected to calibration with the obtained intrinsic frequency set as a calibration parameter; and an assistance step (SP108) in which a movement of the analysis subject 12 is assisted by controlling an operation of the calibrated movement assistance device 18.

As described above, by obtaining the intrinsic frequency correlated with a movement intention or a brain state of the analysis subject 12 on the basis of the frequency characteristic related to the time series of the electroencephalographic signals of the analysis subject 12 acquired during rest, the intrinsic frequency can be identified using the acquired electroencephalographic signals while keeping the analysis subject 12 in the resting state, that is, without requesting an additional movement. Consequently, when the electroencephalographic signals of the analysis subject 12 are analyzed to identify the intrinsic frequency correlated with the movement intention or the brain state, the time required for identification can be significantly reduced.

In addition, the electroencephalogram analysis device 16 may further include the calculation unit 52 that calculates a sample value of a peak frequency in the frequency characteristic, and the estimation unit 54 that obtains an estimate value of the peak frequency using a parent population of the calculated sample value, and the computation unit 58 may transform the estimate value obtained by the estimation unit 54 into the intrinsic frequency according to a preliminarily set transformation rule.

That is, in the electroencephalogram analysis device 16, the estimation unit 54 estimates the estimate value of the peak frequency, the computation unit 58 transforms the estimate value into the intrinsic frequency; and the intrinsic frequency may be thereby obtained without acquiring an electroencephalographic signal of the analysis subject 12 during movement intention or without requesting the analysis subject 12 to move a paralyzed part. Consequently, the mental and physical burden on the analysis subject 12 is significantly reduced.

In addition, the estimation unit 54 may obtain, for each unit period, the estimate value based on the sequential Bayesian method, using a parent population of the sample value accumulated by repeatedly performing acquisition by the signal acquisition unit 40 and calculation by the calculation unit 52 for the unit period from the starting point of the measurement of the electroencephalographic signals.

In addition, when the electroencephalogram analysis device 16 further includes the determination unit 56 that determines, each time the estimation unit 54 performs an estimation, whether a termination condition is met, the estimation unit 54 may terminate the estimation of the peak frequency when the determination unit 56 determines that the termination condition is met. By halting the estimation process when the termination condition is met, the time required to identify the intrinsic frequency can be further reduced.

In addition, when the peak frequency is an alpha frequency (that is, the IAF) within the alpha band, and the intrinsic frequency is an individual SMR-ERD frequency (that is, the ISF), the transformation rule may be expressed as an identity function or a linear function with the IAF estimate value as an argument. Computational speed is improved by using a simpler transformation rule.

In addition, the transformation rule may be determined depending on the analysis subject 12. Consequently, a transformation more suited to the characteristics of the analysis subject 12 becomes possible.

In addition, when the signal acquisition unit 40 acquires the first time series of the electroencephalographic signals of the analysis subject 12 measured during rest or acquires the second time series of the electroencephalographic signals of the analysis subject 12 measured during motor imagery, the computation unit 58 may perform the first computation in which the intrinsic frequency is obtained using only the first time series. Furthermore, the computation unit 58 may perform the first computation or the second computation in which the intrinsic frequency is obtained using both the first time series and the second time series, with the first computation and the second computation switched.

In addition, the electroencephalogram analysis device 16 may further include the presentation unit that presents information for requesting a resting state to the analysis subject 12. In addition, the electroencephalogram analysis device 16 may further include the assistance control unit 44 that controls the movement assistance device 18 for assisting a movement of the analysis subject 12 on the basis of the intrinsic frequency obtained by the computation unit 58. Consequently, analysis on the analysis subject 12 or movement assistance for the analysis subject 12 becomes possible.

[Modification]

Note that the present invention is not limited to the above-described embodiment, and it is needless to say that the present invention can be freely modified without departing from the spirit of the present invention. Alternatively, each configuration may be arbitrarily combined as long as no technical contradiction arises. Alternatively, the execution order of the steps constituting the flowcharts may be changed as long as no technical contradiction arises.

In the above-described embodiment, the case where the electroencephalogram analysis device 16 analyzes electroencephalographic signals and controls the movement assistance device 18 has been described as an example, but the system configuration is not limited thereto. For example, an analysis unit and a control unit may be separately provided, and may be configured to be capable of exchanging necessary data with each other via wired communication or wireless communication. In this case, a cloud server device or an on-premises server device may perform analysis process. In particular, in a case of a cloud server device, the server device may be a group of computers that construct a distributed system.

[Reference Signs List]

10: BMI system (movement assistance system)
12: Analysis subject
14: Electroencephalograph
16: Electroencephalogram analysis device (computer)
18: Movement assistance device
26: Processor
28: Memory
40: Signal acquisition unit (acquisition unit)
42: Frequency identification unit

44: Assistance control unit
50: Preprocessing unit
52: Calculation unit
54: Estimation unit
56: Determination unit
58: Computation unit

**Claims**

1. An electroencephalogram analysis device comprising:

    an acquisition unit that acquires a time series of electroencephalographic signals of an analysis subject; and
    a computation unit that obtains an intrinsic frequency correlated with a movement intention or a brain state of the analysis subject based on a frequency characteristic related to the time series of the electroencephalographic signals of the analysis subject acquired by the acquisition unit during rest.

2. The electroencephalogram analysis device according to Claim 1, further comprising:

    a calculation unit that calculates a sample value of a peak frequency in the frequency characteristic; and
    an estimation unit that obtains an estimate value of the peak frequency using a parent population of the sample value calculated by the calculation unit, wherein
    the computation unit transforms the estimate value obtained by the estimation unit into the intrinsic frequency according to a preliminarily set transformation rule.

3. The electroencephalogram analysis device according to Claim 2, wherein
    the estimation unit obtains the estimate value, and the computation unit transforms the estimate value into the intrinsic frequency; and the intrinsic frequency is thereby obtained without acquiring an electroencephalographic signal of the analysis subject during movement intention.

4. The electroencephalogram analysis device according to Claim 2, wherein
    the estimation unit obtains the estimate value, and the computation unit transforms the estimate value into the intrinsic frequency; and the intrinsic frequency is thereby obtained without requesting the analysis subject to move a paralyzed part.

5. The electroencephalogram analysis device according to Claim 2, wherein
    the estimation unit obtains, for each unit period, the estimate value based on a sequential Bayesian method, using a parent population of the sample value accumulated by repeatedly performing acquisition by the acquisition unit and calculation by the calculation unit for the unit period from a starting point of measurement of the electroencephalographic signals.

6. The electroencephalogram analysis device according to Claim 4, further comprising

    a determination unit that determines, each time the estimation unit performs an estimation, whether a termination condition is met, wherein
    the estimation unit terminates the estimation of the peak frequency when the determination unit determines that the termination condition is met.

7. The electroencephalogram analysis device according to Claim 2, wherein

    the peak frequency is an alpha frequency within an alpha band,
    the intrinsic frequency is an individual SMR-ERD frequency, and
    the transformation rule is expressed as an identity function or a linear function with the estimate value as an argument.

8. The electroencephalogram analysis device according to Claim 2, wherein
    the transformation rule is determined depending on the analysis subject.

9. The electroencephalogram analysis device according to Claim 1, wherein

the acquisition unit acquires either a first time series of the electroencephalographic signals of the analysis subject measured during rest or a second time series of the electroencephalographic signals of the analysis subject measured during motor imagery, and

the computation unit performs a first computation in which the intrinsic frequency is obtained using only the first time series.

10. The electroencephalogram analysis device according to Claim 9, wherein

the computation unit performs the first computation or a second computation in which the intrinsic frequency is obtained using both the first time series and the second time series, with the first computation and the second computation switched.

11. The electroencephalogram analysis device according to Claim 1, further comprising

a presentation unit that presents information for requesting a resting state to the analysis subject.

12. The electroencephalogram analysis device according to Claim 1, further comprising

an assistance control unit that controls a movement assistance device for assisting a movement of the analysis subject based on the intrinsic frequency obtained by the computation unit.

13. An electroencephalogram analysis program causing one or more computers to execute:

an acquisition step of acquiring a time series of electroencephalographic signals of an analysis subject; and

a computation step of obtaining an intrinsic frequency correlated with a movement intention or a state of the analysis subject based on a frequency characteristic related to the time series of the electroencephalographic signals of the analysis subject acquired during rest.

14. A movement assistance system comprising:

the electroencephalogram analysis device according to Claim 12;

an electroencephalograph that supplies, to the electroencephalogram analysis device, an electroencephalographic signal obtained by measuring an electroencephalogram of an analysis subject; and

a movement assistance device that assists a movement of the analysis subject as the movement assistance device operates according to control performed by the electroencephalogram analysis device.

15. A movement assistance method using a system including an electroencephalograph that measures an electroencephalogram of an analysis subject and outputs electroencephalographic signals, an electroencephalogram analysis device that analyzes the electroencephalographic signals supplied from the electroencephalograph, and a movement assistance device that operates according to control performed by the electroencephalogram analysis device to assist a movement of the analysis subject, the method executing:

an acquisition step in which the electroencephalogram analysis device measures an electroencephalogram of the analysis subject during rest using the electroencephalograph to acquire a time series of the electroencephalographic signals;

a computation step in which the electroencephalogram analysis device obtains an intrinsic frequency correlated with a movement intention or a brain state of the analysis subject based on a frequency characteristic related to the acquired time series of the electroencephalographic signals;

a calibration step in which the movement assistance device is subjected to calibration with the obtained intrinsic frequency set as a calibration parameter; and

an assistance step in which a movement of the analysis subject is assisted by controlling an operation of the calibrated movement assistance device.

# FIG.1

10

16

ELECTROENCEPHALOGRAM ANALYSIS DEVICE

22
OPERATION UNIT

23
PRESENTATION UNIT

24
SENSOR CONT-ROLLER

26
PROCESSOR

28
MEMORY

EEG SIGNALS

14
ELECTRO-ENCEPHALO-GRAPH

12

12h

18
MOVEMENT ASSISTANCE DEVICE

12a

# FIG.2

PROCESSOR

26

SIGNAL ACQUISITION UNIT — 40

FREQUENCY
IDENTIFICATION UNIT — 42

PREPROCESSING
UNIT — 50

CALCULATION
UNIT — 52

ESTIMATION UNIT — 54

DETERMINATION
UNIT — 56

COMPUTATION
UNIT — 58

ASSISTANCE CONTROL UNIT — 44

SETTING UNIT — 60

DECISION UNIT — 62

MEMORY

28

ANALYSIS SUBJECT
INFORMATION — 70

FREQUENCY
INFORMATION — 72

TRANSFORMATION
INFORMATION — 74

# FIG.3

```
              ┌─────────────┐
              │    START    │
              └──────┬──────┘
                     │
                     ▼
SP100      ┌─────────────────────┐
           │       ATTACH        │
           │ ELECTROENCEPHALOGRAPH│
           └──────────┬──────────┘
                      │
                      ▼
SP102      ┌─────────────────────┐
           │   START MONITORING  │
           │ ELECTROENCEPHALOGRAM│
           └──────────┬──────────┘
                      │
                      ▼
SP104    ╱─────────────────────────╲     NO
         │  ANALYSIS SUBJECT HAS    ├──────┐
         │  BEEN IN RESTING STATE?  │      │
         ╲─────────────────────────╱      │
                      │ YES                │
                      ▼                    │
SP106      ┌─────────────────────┐         │
           │  EXECUTE CALIBRATION │         │
           │(MAINTAIN RESTING STATE)│        │
           └──────────┬──────────┘         │
                      │                    │
                      ▼                    │
SP108      ┌─────────────────────┐         │
           │       PROVIDE        │         │
           │  NEUROREHABILITATION │         │
           └──────────┬──────────┘         │
                      │                    │
                      ▼                    │
              ┌─────────────┐              │
              │     END     │              │
              └─────────────┘              │
```

# FIG.4

SP106

START

SP10 — ACQUIRE ELECTROENCEPHALO-GRAPHIC SIGNALS WITHIN UNIT PERIOD

SP12 — EXECUTE PREPROCESSING

SP14 — CALCULATE IAF SAMPLE VALUE

SP16 — ESTIMATION TIMING HAS ARRIVED?  — NO

YES

SP18 — ESTIMATE IAF USING SEQUENTIAL BAYESIAN METHOD

SP20 — TERMINATION CONDITION IS MET? — NO

YES

SP22 — TRANSFORMATION FROM IAF TO ISF

SP24 — SET ISF(CALIBRATION OF MOVEMENT ASSISTANCE DEVICE)

RETURN

# FIG.5

# FIG.6

# FIG.7

ESTIMATION
ERROR
[Hz]

+2σ BOUNDARY LINE

1Hz

0

AVERAGE

−2σ BOUNDARY LINE

-2

-4

-6

0          50          100          150

TIME
[s]

# FIG.8

# FIG.9

# FIG.10

# FIG.11

# FIG.12

DURING REST | DURING MOTOR IMAGERY

FREQUENCY [Hz]

30

25

20

15

10

5

-4    -2    0    2    4

TIME [s]

LOW INTENSITY BAND

ISF

## INTERNATIONAL SEARCH REPORT

| | International application No. |
|---|---|
| | **PCT/JP2023/038668** |

| A. | CLASSIFICATION OF SUBJECT MATTER |
|---|---|

*A61B 5/374*(2021.01)i; *A61B 5/16*(2006.01)i
FI:   A61B5/374; A61B5/16 200

According to International Patent Classification (IPC) or to both national classification and IPC

| B. | FIELDS SEARCHED |
|---|---|

Minimum documentation searched (classification system followed by classification symbols)

A61B5/374; A61B5/16

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); PubMed

| C. | DOCUMENTS CONSIDERED TO BE RELEVANT |
|---|---|

| Category* | Citation of document, with indication, where appropriate, of the relevant passages | Relevant to claim No. |
|---|---|---|
| A | MAGNUSON, J. R. et al. Low-frequency neural activity at rest is correlated with the movement-related cortical potentials elicited during both real and imagined movements. Neuroscience Letters. 18 January 2021, vol. 742, article no.135530, pp. 1-6, <DOI: 10.1016/j.neulet.2020.135530>  abstract | 1-15 |
| A | WO 2021/070456 A1 (VIE STYLE, INC.) 15 April 2021 (2021-04-15)  paragraphs [0066]-[0068] | 1-15 |
| A | WO 2019/078325 A1 (PANASONIC CORPORATION) 25 April 2019 (2019-04-25)  paragraphs [0011]-[0143] | 1-15 |
| P, X | WO 2022/236342 A1 (WAVE NEUROSCIENCE, INC.) 10 November 2022 (2022-11-10)  abstract, paragraphs [0006], [0037], [0052]-[0062], fig. 1 | 1, 9, 13 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **19 December 2023** | **09 January 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2023/038668**

| Patent document cited in search report | | | Publication date (day/month/year) | Patent family member(s) | | | Publication date (day/month/year) |
|---|---|---|---|---|---|---|---|
| WO | 2021/070456 | A1 | 15 April 2021 | US paragraphs [0097]-[0100] | 2022/0386919 | A1 | |
| WO | 2019/078325 | A1 | 25 April 2019 | US paragraphs [0022]-[0163] | 2020/0253499 | A1 | |
| | | | | EP | 3698712 | A1 | |
| WO | 2022/236342 | A1 | 10 November 2022 | US | 2022/0400995 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **B. BLANKERTZ et al.** Neurophysiological predictor of SMR-based BCI performance. *NeuroImage*, 2010, vol. 51, 1303-1309